# FASCICULE DE BREVET EUROPEEN

(11) **EP 1 246 565 B1**
(45) Date de publication et mention de la délivrance du brevet: **19.10.2005**
(21) Numéro de dépôt: 01903901.5
(22) Date de dépôt: 10.01.2001
(51) Int. Cl.: A61B 5/103, A61B 6/00

(54) **DISPOSITIF D'EVALUATION DE LA POSITION D'EQUILIBRE DU CORPS HUMAIN**
AUSWERTUNGSEINRICHTUNG DES GLEICHGEWICHTSTANDES DES MENSCHLICHEN KÖRPERS
DEVICE FOR EVALUATING A POSITION OF BALANCE FOR THE HUMAN BODY

(30) Priorité: 10.01.2000 FR 0000497
(43) Date de publication de la demande: 09.10.2002
(73) Titulaire: SDGI Holdings, Inc., Wilmington, DE 19801 (US)
(72) Inventeur: BERTHONNAUD,Eric, 69260 Charbonnières Les Bains (FR); DIMNET,Joannès, 69300 Caluire et Cuire (FR); ROUSSOULY,Pierre, 69450 Saint Cyr au Mont d'Or (FR)
(74) Mandataire: Jacobson, Claude
(86) Numéro de dépôt international: PCT/FR2001/000060
(87) Numéro de publication internationale: WO 2001/050956

(56) Documents cités:
- EP-A- 0 119 660
- NL-A- 7 415 910
- US-A- 5 609 162

## Description

### DOMAINE TECHNIQUE DE L'INVENTION

La présente invention concerne un dispositif d'évaluation d'une position d'équilibre du corps humain, notamment d'une position d'équilibre de la colonne vertébrale.

Le diagnostic des douleurs vertébrales repose généralement sur l'interprétation d'une ou plusieurs radiographies, réalisées à l'aide d'un appareil comprenant une source de rayons X, des moyens supports de plaque cible sensible aux rayons X, des moyens supports de patient adaptés pour supporter un patient en position fixe entre la source de rayons X et les moyens supports de plaque cible. Le praticien doit interpréter les radiographies, pour en déduire les causes probables des douleurs vertébrales du patient.

L'analyse des radiographies est une opération délicate, et, malgré la grande expérience des praticiens, une telle analyse ne permet pas de rendre compte de façon suffisamment fiable et complète des causes d'apparition de certaines affections vertébrales. Et dans certains cas, les radiographies conduisent à des erreurs de diagnostic.

Pour effectuer une analyse plus complète du squelette humain, dans le but de mieux connaître l'origine de certaines douleurs du dos des patients, on a déjà proposé, dans les documents NL 7 415 910 A et EP 0 119 660 A, de tracer de façon automatique sur un cliché radiographique de la colonne vertébrale l'axe vertical passant par le centre de gravité du patient. Un dispositif décrit dans ces documents comprend pour cela une source de rayons X, des moyens supports de plaque cible sensible aux rayons X, des moyens supports de patient adaptés pour supporter un patient en position fixe entre la source de rayons X et les moyens supports de plaque cible, et des moyens associés aux moyens supports de patient pour détecter la position horizontale de l'axe global de gravité du patient lors du fonctionnement de la source de rayons X et pour générer des signaux de position. Les signaux de position commandent un chariot supérieur en déplacement transversal et soutenant un fil de plomb vertical placé entre le patient et le support de plaque cible sensible aux rayons X, visant à placer le fil en alignement avec la source de rayons X et l'axe global de gravité. On peut ainsi visualiser la position de l'axe global de gravité du patient sur les clichés radiographiques, caractérisant l'équilibre du patient.

Un tel dispositif présente toutefois plusieurs inconvénients qui empêchent en pratique son utilisation effective. Tout d'abord, la précision et la fiabilité de position de l'image radiographique de l'axe global de gravité ne sont pas satisfaisantes. Et surtout, le système mécanique à chariot mobile et fil vertical est une source de gêne et d'erreurs par son encombrement, par les risques d'accrochage du fil, et par l'attention qu'il faut nécessairement porter au temps de déplacement du chariot porte-fil.

Un autre inconvénient résulte généralement de la position variable et non contrôlée du patient sur les moyens supports de patient, ce qui nuit à la lisibilité du cliché radiographique.

### EXPOSE DE L'INVENTION

Le problème proposé par la présente invention est de concevoir un nouveau dispositif permettant d'éviter les inconvénients des dispositifs connus, notamment en supprimant toutes sources de gêne et d'erreurs pouvant résulter de l'utilisation d'un fil vertical mobile, et en rendant totalement transparent pour l'utilisateur le transfert de la position de l'axe global de gravité du patient sur l'image radiographique.

Un autre problème de l'invention est d'améliorer la précision et la fiabilité des images radiographiques ainsi obtenues, de façon à permettre une interprétation fine de la position relative de l'axe global de gravité par rapport au squelette du patient.

Pour atteindre ces objets ainsi que d'autres, l'invention prévoit un dispositif d'évaluation d'une position d'équilibre du corps humain, comprenant une source de rayons X et des moyens supports de plaque cible sensible aux rayons X pour réaliser sur la plaque cible une image radiographique du patient, des moyens supports de patient adaptés pour supporter un patient en position fixe entre la source de rayons X et les moyens supports de plaque cible et pour générer des signaux de position image de la position horizontale de l'axe global de gravité du patient par rapport à la source de rayons X et à la plaque cible ;

le dispositif selon l'invention comprend :
- des moyens pour numériser par balayage l'image radiographique du patient réalisée sur la plaque cible, et pour générer ainsi une image numérisée,
- des moyens pour générer, en fonction desdits signaux de position, une image numérisée de l'ombre portée de l'axe global de gravité dans le plan de la plaque cible,
- des moyens pour combiner à ladite image radiographique numérisée du patient l'image numérisée de l'ombre portée de l'axe global de gravité, et pour générer ainsi une image numérisée combinée,
- et des moyens pour visualiser ladite image numérisée combinée.

L'image est ainsi réalisée par des moyens électroniques situés entièrement en dehors de l'espace occupé par le patient lors de la prise de radiographie, et totalement insensibles aux risques de déplacement par les mouvements du patient. De plus, la précision de l'image ainsi obtenue est sensiblement améliorée, puisqu'on élimine toute erreur de position du fil.

Dans le dispositif selon l'invention, les moyens pour générer une image numérisée de l'ombre portée de l'axe global de gravité comprennent :
- une unité de calcul et une mémoire associée,
- des données géométriques, enregistrées dans la mémoire, et correspondant aux positions relatives de la source de rayons X, du plan de la plaque sensible, et des moyens supports de patient dans un repère fixe,
- un programme enregistré dans une zone de mémoire de programme de la mémoire, adapté pour mémoriser lesdits signaux de position à l'instant de prise d'image radiographique, et adapté pour calculer en fonction desdits signaux de position la droite intersection du plan de la plaque sensible et du plan vertical passant par la source de rayons X et par la position horizontale de l'axe global de gravité, et pour mémoriser le résultat de ce calcul qui constitue l'image de l'axe global de gravité.

De préférence, le dispositif est tel que :
- il comprend un scanner pour scruter par balayage l'image radiographique réalisée sur la plaque cible sensible aux rayons X et pour produire une suite de signaux numériques constituant l'image radiographique numérisée,
- le programme est adapté pour assurer la réception desdits signaux numériques et pour les enregistrer dans la mémoire,
- le programme est adapté pour modifier l'image radiographique numérisée enregistrée en substituant l'image de l'axe global de gravité, générant ainsi ladite image numérisée combinée,
- et le programme est adapté pour afficher ladite image numérisée combinée sur un moniteur, ou pour produire son impression sur un support.

Selon un mode de réalisation préféré, améliorant encore la précision, le programme comprend une séquence de calibrage, adaptée pour scruter l'image radiographique d'un objet de calibrage à repères radio-opaques et pour calculer, à partir des dimensions et positions connues des repères radio-opaques dudit objet de calibrage, lesdites données géométriques, et pour les enregistrer dans la mémoire. Cette disposition permet de résoudre le problème de l'incertitude liée à la position inconnue de la source de rayons X qui est généralement incorporée à l'intérieur d'une tête d'émission dont on ne distingue pas l'intérieur. En positionnant de façon appropriée et repérée l'objet de calibrage sur les moyens supports de patient, et en prenant un cliché radiographique de l'objet de calibrage ainsi positionné, on détermine en une seule fois toutes les données géométriques nécessaires, c'est-à-dire les positions relatives de la source, des moyens supports de patient et de la plaque cible, permettant ensuite un calcul précis de la position relative de l'image de l'axe global de gravité par rapport à l'image radiographique des os du patient.

L'une des causes du manque de précision et de fiabilité des dispositifs connus qui comprennent un plateau de force détectant la position horizontale de l'axe global de gravité du patient est la dérive possible des caractéristiques des capteurs du plateau de force. Il résulte de cette dérive que les signaux produits par chacun des capteurs peuvent varier dans le temps, ce qui conduit à une détermination erronée de la position de l'axe global de gravité. L'invention permet de résoudre ce problème, en prévoyant un poids d'étalonnage et une séquence d'étalonnage à effectuer périodiquement. Le poids d'étalonnage, amovible et positionnable sur les moyens supports de patient, présente une forme déterminée, comprend des moyens de repérage de position à mettre en coïncidence avec des moyens de repérage complémentaires des moyens supports de patient, et comprend un centre de gravité dont la position relative est connue par rapport aux moyens de repérage de position. La séquence d'étalonnage, prévue dans le programme enregistré, est adaptée pour calculer les paramètres de correction des signaux de force produits par chaque capteur, de façon à amener en coïncidence le centre de gravité calculé avec la position géométrique connue du centre de gravité réel du poids d'étalonnage. Les paramètres de correction sont alors enregistrés en mémoire pour permettre les calculs ultérieurs fiables de position de l'axe global de gravité des patients.

De préférence, pour améliorer la précision, le repère fixe est centré sur le plan vertical passant par la source de rayons X et perpendiculaire aux moyens supports de plaque cible sensible aux rayons X.

De préférence, les moyens supports de patient comprennent des appuis-bras sur lesquels le patient peut appuyer ses bras dans une position définie avancée.

Dans un mode de réalisation avantageux, les moyens supports de patient sont montés rotatifs autour d'un axe vertical médian, permettant d'orienter angulairement le patient par rapport à la direction entre la source de rayons X et les moyens supports de plaque cible.

Selon une réalisation préférée, les appuis-bras sont solidaires d'un plateau tournant qui lui-même porte un plateau de force de balance modifiée par une pluralité de capteurs de force répartis selon des positions repérées par rapport au repère et produisant des signaux de force utilisés par l'unité de calcul et le programme comme signaux de position.

De préférence, le dispositif comprend des moyens d'indexation pour fixer sélectivement la position angulaire des moyens supports de patient selon au moins tous les 45°.

### DESCRIPTION SOMMAIRE DES DESSINS

D'autres objets, caractéristiques et avantages de la présente invention ressortiront de la description suivante de modes de réalisation particuliers, faite en relation avec les figures jointes, dans lesquelles :
- la figure 1 représente schématiquement et en perspective un dispositif d'évaluation selon un mode de réalisation de la présente invention ;
- la figure 2 est une vue de face en coupe diamétrale des moyens supports de patient dans le dispositif d'évaluation de la figure 1 ;
- la figure 3 illustre en vue de dessus le dispositif d'évaluation de la figure 1 ;
- la figure 4 est une vue en perspective illustrant l'étape de calibrage à l'aide d'un objet de calibrage ; et
- la figure 5 est un schéma bloc simplifié illustrant les éléments essentiels de matériel et de logiciel permettant les calculs pour la génération des images selon l'invention.

### DESCRIPTION DES MODES DE REALISATION PREFERES

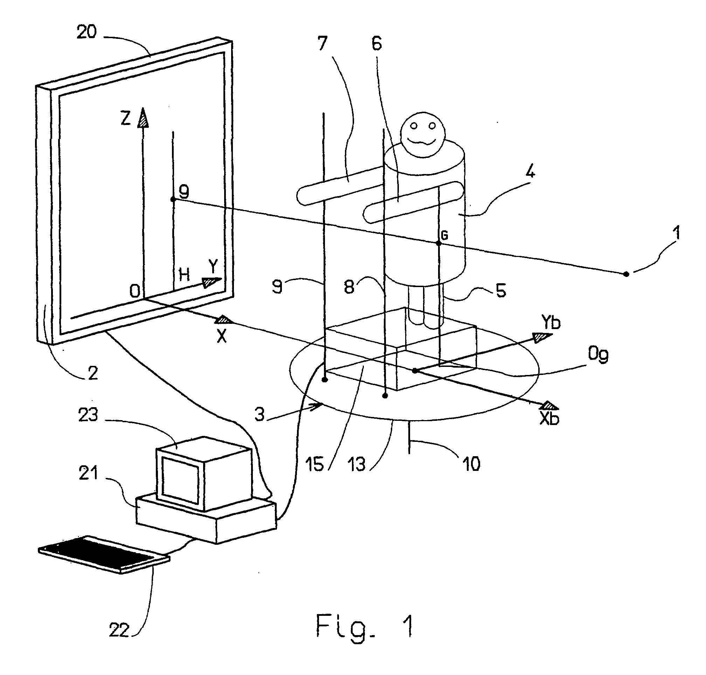

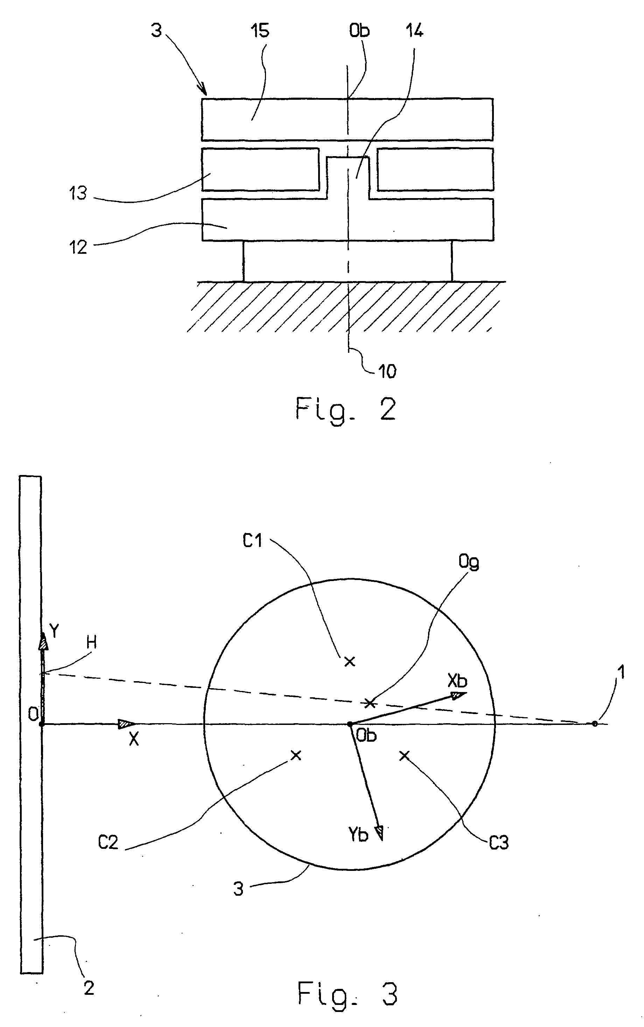

Dans le mode de réalisation illustré sur les figures 1 et 3, le dispositif comprend une source de rayons X 1, des moyens supports de plaque cible 2 pour supporter une plaque cible sensible aux rayons X et orientée verticalement à une certaine distance horizontale de la source de rayons X 1, et des moyens supports de patient 3 adaptés pour supporter un patient 4 en position fixe entre la source de rayons X 1 et les moyens supports de plaque cible 2. Le dispositif selon l'invention est une installation pour la prise de radiographies. Autrement dit, la source de rayons X 1, et les moyens supports de plaque cible 2 peuvent avoir une structure telle que celles habituellement utilisées dans les installations de radiographie médicales.

Selon l'invention, les moyens supports de patient 3 comprennent des moyens pour détecter la position horizontale, c'est-à-dire les coordonnées dans le plan horizontal, de l'axe global de gravité 5 du patient ou axe vertical passant par le centre de gravité G du patient 4, lors du fonctionnement de la source de rayons X 1.

A priori, l'invention peut s'appliquer à l'analyse de l'équilibre de patients 4 dans diverses positions telles que la position debout, ou la position assise par exemple. Sur la figure 1, on a représenté le dispositif selon l'invention dans le mode de réalisation dans lequel les moyens supports de patient 3, les moyens de source de rayons X 1 et les moyens supports de plaque cible 2 sont conformés pour réaliser une radiographie et un repérage simultané de l'axe global de gravité 5 d'un patient 4 en position debout.

Dans cette position debout, pour l'analyse de la colonne vertébrale, le dispositif est agencé pour prendre une radiographie de la zone thoracique, de la zone lombaire, de la zone pelvienne et de la zone fémorale supérieure du patient 4.

Dans le mode de réalisation dans lequel le patient 4 est en position debout, il est important que les bras 6 et 7 du patient aient une position définie constante, car leur position influe sur celle du centre de gravité G et sur la qualité de l'image radiographique obtenue. Par exemple, comme illustré sur la figure, les moyens supports de patient 3 comprennent des appuis-bras 8 et 9 tels que des tiges verticales, sur lesquels le patient 4 peut appuyer ses bras 6 et 7 en position avancée, par exemple en position horizontale, agrippant les tiges 8 et 9 dans ses mains.

De préférence, les moyens supports de patient 3 sont montés rotatifs autour d'un axe vertical médian 10, permettant d'orienter angulairement le patient 4 par rapport à la direction de propagation des rayons entre la source de rayons X 1 et les moyens supports de plaque cible 2, selon plusieurs positions angulaires successives dans lesquelles plusieurs radiographies peuvent être prises, par exemple de face, puis de profil. Des moyens d'indexation permettent de fixer sélectivement la position angulaire des moyens supports de patient 3 autour de l'axe vertical médian 10 selon au moins huit positions réparties tous les 45°.

Dans le mode de réalisation avantageux illustré sur les figures 2 et 3, le patient 4 repose sur des moyens supports de patient 3 comprenant un plateau de force de balance modifiée 15 muni d'une pluralité de capteurs de force tels que les capteurs C1, C2 et C3, répartis selon des positions repérées par rapport à un repère XbObYb, et associés à des moyens de calcul pour calculer la position Og, dans le plan horizontal, de la résultante des forces dans ledit repère XbObYb. On peut naturellement prévoir un nombre de capteurs supérieur à trois.

Le repère XbObYb peut avantageusement être centré en Ob sur le plan XOZ vertical passant par la source de rayons X 1 et perpendiculaire aux moyens supports de plaque cible 2 sensible aux rayons X.

Par exemple, comme illustré en vue de face en coupe sur la figure 2, les moyens supports de patient 3 comprennent un plateau fixe 12, qui peut être implanté dans le sol, sur lequel est monté un plateau tournant 13 qui tourillonne autour d'un pivot supérieur 14 du plateau fixe 12. Le pivot supérieur 14 définit ainsi l'axe de rotation du plateau tournant 13, qui est également l'axe vertical médian 10 des moyens supports de patient 3.

Le plateau tournant 13 porte un plateau de force de balance modifiée 15, utilisé comme surface d'appui du patient pendant le fonctionnement du dispositif d'évaluation de l'invention. Par exemple, le plateau de force de balance modifiée 15 peut avoir une forme de disque, comme on le voit sur la figure 3.

La plaque constituant le plateau de force de balance modifiée 15 est reliée au plateau tournant 13 par l'intermédiaire d'au moins trois capteurs de force C1, C2 et C3, adaptés pour évaluer la force verticale d'appui entre le plateau de force de balance modifiée 15 et le plateau tournant 13. Par exemple, comme illustré sur la figure 3, les capteurs de force C1, C2 et C3 sont répartis à 120° les uns des autres et à équidistance autour du centre Ob.

Les trois capteurs C1, C2 et C3 sont sollicités par des forces verticales F1, F2 et F3.

Le barycentre des emplacements des capteurs C1, C2 et C3 affectés des coefficients F1, F2 et F3 est le point O_{g} où l'axe global de gravité 5 du patient coupe le plan d'appui. Ce point O_{g} est déterminé par un calcul élémentaire à partir des coordonnées connues des positions des capteurs C1, C2 et C3 et des valeurs des forces F1, F2 et F3 mesurées. Le calcul applique la relation :

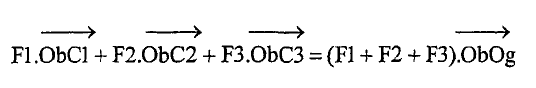

Ce calcul peut être réalisé par un microcontrôleur incorporé dans le plateau de force de balance modifiée 15, ou disposé dans un autre emplacement, et programmé d'une façon appropriée qui est à la portée de l'homme du métier.

De préférence, comme illustré sur la figure 1, on prévoit une unité de calcul 21 et une mémoire associée, par exemple un micro-ordinateur et ses périphériques habituels tels qu'un clavier 22 et un moniteur 23. L'unité de calcul 21 est connectée électriquement au moyen support de patient 3 pour recevoir les signaux de position générés par le moyen support de patient 3 et pour réaliser le calcul des coordonnées du point Og.

Selon l'invention, on prévoit en outre des moyens 20 pour numériser par balayage l'image radiographique du patient sur les moyens supports de plaque cible 2. On peut pour cela utiliser un scanner 20 adapté pour scruter par balayage l'image radiographique réalisée sur la plaque cible 2 sensible aux rayons X, et pour produire une suite de signaux numériques constituant l'image radiographique numérisée. Selon l'invention, on prévoit en outre des moyens de calcul et de traitement d'image pour superposer à ladite image radiographique numérisée du patient l'image Hg numérisée de l'axe global de gravité 5 dans le plan vertical de la plaque sensible aux rayons X.

De préférence, les moyens de calcul sont associés à des moyens de mémorisation assurant la mémorisation des coordonnées de la position Og de la résultante des forces sur le plateau de force de balance modifiée 15 à l'instant de prise de la radiographie. Ainsi, la position relative de l'image radiographique du patient et de l'image Hg de l'axe global de gravité 5 correspond exactement, en tenant compte du grandissement résultant du principe même de la radiographie, à la position relative entre le squelette du patient 4 et la position de son axe global de gravité 5 dans l'attitude définie de prise de radiographie.

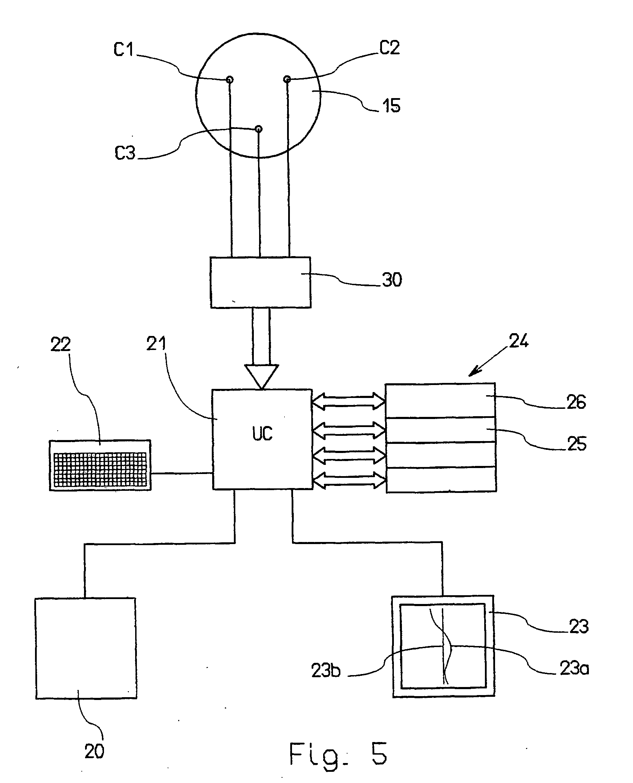

La figure 5 illustre plus en détail le diagramme schématique des éléments permettant les calculs et la génération des images.

On retrouve l'unité de calcul 21 telle que l'unité centrale d'un micro-ordinateur ou d'un microprocesseur, associée à ses périphériques tels que le clavier 22 et le moniteur 23. Les signaux de position générés par les capteurs de force C1, C2 et C3 sont envoyés à l'unité de calcul 21 par l'intermédiaire d'un convertisseur analogique/numérique 30. L'unité de calcul 21 effectue le calcul précédemment mentionné pour en déduire les coordonnées de l'axe global de gravité 5 dans le repère horizontal XbObYb fixe, ou position du point Og. Ces coordonnées sont enregistrées dans une zone de mémoire de données 25 de la mémoire 24 elle-même connectée à l'unité de calcul 21.

La zone de mémoire de données 25 contient en outre les autres données géométriques correspondant aux positions relatives de la source de rayons X 1, du plan de la plaque sensible 2 et des moyens supports de patient 3 dans le repère XbObYb.

Un programme est enregistré dans une zone de mémoire de programme 26 de la mémoire 24. Le programme est adapté pour mémoriser lesdits signaux de position à l'instant de prise d'image radiographique, instant qui est communiqué à l'unité de calcul 21 par une entrée connectée à l'appareil de radiographie. Le programme est adapté pour calculer, en fonction desdits signaux de position ou coordonnées du point Og, la droite Hg intersection du plan de la plaque sensible 2 et du plan vertical passant par la source de rayons X 1 et la position horizontale Og de l'axe global de gravité 5. Le résultat du calcul est mémorisé dans la mémoire 24, et ce résultat de calcul constitue l'image de l'axe global de gravité 5.

Simultanément ou en différé, le scanner 20 scrute par balayage l'image radiographique réalisée sur la plaque cible 2 sensible aux rayons X, et produit une suite de signaux numériques constituant l'image radiographique numérisée. Le programme assure la réception de ces signaux numériques par l'unité de calcul 21, et leur enregistrement dans la mémoire 24.

Le programme est également adapté pour modifier l'image radiographique mémorisée enregistrée dans la mémoire 24 en substituant, dans l'image radiographique numérisée, les points correspondants de l'image de l'axe global de gravité Hg, pour générer ainsi une image numérisée combinée.

Enfin, le programme est adapté pour afficher ladite image numérisée combinée sur le moniteur 23, ou pour produire son impression sur un support. On a ainsi illustré schématiquement sur le moniteur 23 l'image numérisée combinée, comprenant l'image radiographique numérisée 23a et l'image de l'axe global de gravité 23b.

En pratique, il n'est pas rare que se produisent certains déplacements entre la source de rayons X 1 et le support de plaque cible 2, ou que l'on ne connaisse pas de façon précise la position exacte de la source de rayons X 1 qui est généralement cachée dans une tête émettrice. Il n'est alors pas possible de faire un calcul précis de la position exacte de l'image Hg de l'axe global de gravité 5.

Pour résoudre ce problème, on peut prévoir des moyens de calibrage qui seront décrits ci-après.

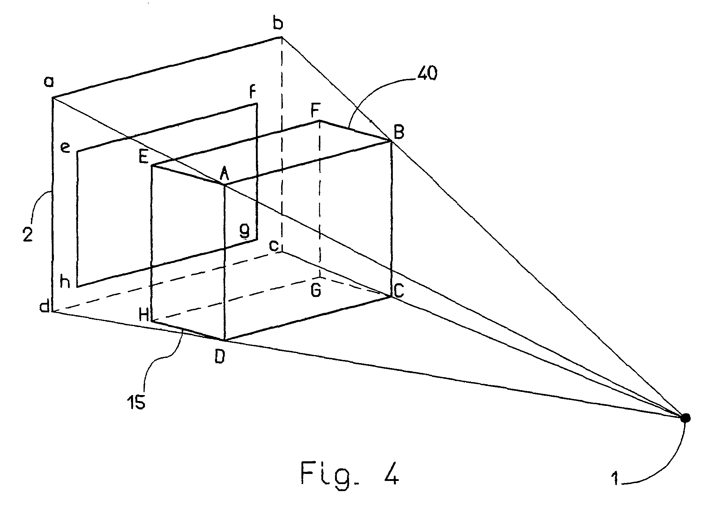

Comme illustré sur la figure 4, le calibrage est effectué au cours d'une séquence de calibrage dans laquelle on réalise une image radiographique d'un objet de calibrage 40 de dimensions connues et de structure particulière, que l'on place en une position repérée et appropriée sur les moyens supports de patient 3. L'objet de calibrage 40 est avantageusement un parallélépipède de matière plastique radiotransparente, par exemple de dimensions 180 mm × 180 mm × 300 mm, dans lequel huit billes de plomb radio-opaques de diamètre 3 mm sont implantées aux sommets. L'objet de calibrage 40 porte un repère matérialisé sur sa base inférieure, et on met ce repère en coïncidence parfaite avec un repère correspondant des moyens supports de patient 3.

L'objet de calibrage est radiographié dans les mêmes conditions que le patient, comme illustré sur la figure 4, en plaçant une plaque cible 2 en même position que les radios à effectuer ultérieurement.

On effectue ensuite la numérisation de l'image radiographique obtenue, à l'aide du scanner 20, et le programme comporte une séquence de programme adaptée pour reconnaître les formes rondes constituant les images des billes de l'objet de calibrage 40. Par exemple, pour un parallélépipède ayant des billes à ses huit sommets A, B, C, D, E, F , G et H, on obtient une image abcd pour la face avant, et une image plus petite efgh pour la face arrière.

Sont inconnues les trois coordonnées de la source de rayons X 1 par rapport au repère fixe XbObYb, les trois coordonnées du centre de l'objet de calibrage 40 par rapport au repère fixe, et deux paramètres angulaires représentant les inclinaisons possibles de l'objet de calibrage 40 par rapport au repère XbObYb. A ces huit inconnues correspondent les seize coordonnées mesurées des huit images abcd, efgh des billes. Le programme est adapté pour mesurer sur l'image numérisée les coordonnées de ces points abcdefgh. Une technique numérique à la portée de l'homme du métier est utilisée pour résoudre le système comportant seize équations avec huit inconnues. La redondance, résultant du nombre supérieur d'équations par rapport aux inconnues, est utilisée pour améliorer la précision des calculs. Ainsi le programme en déduit les données géométriques qui sont alors enregistrées dans la zone de mémoire de données 25 de la mémoire 24.

En pratique, il n'est pas rare que les capteurs de force C1, C2 et C3, qui sont des capteurs électroniques générant des signaux électriques, présentent des caractéristiques techniques qui dérivent dans le temps et produisent donc des signaux de force dont le traitement ne permet plus de restituer la position exacte de l'axe de gravité global des patients.

Ainsi, dans le cas où les moyens supports de patient comprennent un plateau de force porte-patient porté par une pluralité de capteurs de force, on peut avantageusement prévoir de réaliser périodiquement un réétalonnage du dispositif, en utilisant un poids d'étalonnage et une séquence d'étalonnage.

Le poids d'étalonnage est un poids amovible et positionnable sur les moyens supports de patient 3. Le poids d'étalonnage a une forme déterminée, par exemple une forme parallélépipédique comme l'objet de calibrage 40, ou une forme cylindrique, et il présente un centre de gravité dont la position géométrique est connue dans le volume du poids d'étalonnage. Le poids d'étalonnage comprend des moyens de repérage de position, par exemple sur sa face inférieure, moyens de repérage que l'on met en coïncidence avec des moyens de repérage complémentaires prévus sur les moyens supports de patient 3. Ainsi, la position géométrique du centre de gravité du poids d'étalonnage est connue par rapport aux moyens supports de patient 3, et donc par rapport au repère fixe XbObYb.

Le programme enregistré dans la mémoire comprend une séquence d'étalonnage, adaptée pour calculer les paramètres de correction des signaux de force produits par les capteurs C1, C2 et C3 de façon à amener en coïncidence le centre de gravité calculé avec la position géométrique connue du centre de gravité réel du poids d'étalonnage. Les paramètres de correction sont alors enregistrés en mémoire, pour permettre ensuite les calculs ultérieurs fiables de position de l'axe global de gravité des patients.

On comprend que le dispositif selon l'invention permet une meilleure compréhension du mécanisme de l'équilibre sagittal. Les risques d'erreur sont minimisés, car le dispositif donne de façon automatique et précise la position relative de l'axe global de gravité 5 et du squelette humain.

Dans le mode de réalisation pour prise de radiographies en position debout, l'invention permet de prendre en compte les membres inférieurs, et de mieux interpréter les défauts de courbure de la colonne vertébrale.

A partir de l'image radiographique complétée de l'image Hg de l'axe global de gravité 5, le praticien peut mesurer l'écart entre l'axe global de gravité 5 et l'axe des têtes fémorales. Il peut également repérer les centres des têtes fémorales pour évaluer l'écart antéropostérieur entre l'axe global de gravité 5 et les têtes fémorales.

Après marquage sur la radiographie de points de repère spécifiques par un opérateur, le dispositif peut également, à l'aide d'un ordinateur programmé de façon appropriée, reconstruire un modèle vertébral et pelvien permettant de caractériser et d'évaluer au mieux les courbures des différents tronçons de la colonne vertébrale.

La présente invention n'est pas limitée aux modes de réalisation qui ont été explicitement décrits, mais elle en inclut les diverses variantes et généralisations contenues dans le domaine des revendications ci-après.

## Revendications

1. Dispositif d'évaluation d'une position d'équilibre du corps humain, comprenant une source de rayons X (1) et des moyens supports de plaque cible (2) sensible aux rayons X pour réaliser sur la plaque cible (2) une image radiographique du patient, des moyens supports de patient (3) adaptés pour supporter un patient (4) en position fixe entre la source de rayons x (1) et les moyens supports de plaque cible (2) et pour générer des signaux de position image de la position horizontale de l'axe global de gravité (5) du patient (4) par rapport à la source de rayons X (1) et à la plaque cible (2),
**caractérisé en ce qu'**il comprend :
- des moyens (20) pour numériser par balayage l'image radiographique du patient réalisée sur la plaque cible (2), et pour générer ainsi une image radiographique numérisée,
- des moyens (21) pour générer, en fonction desdits signaux de position, une image numérisée de l'ombre portée (Hg) de l'axe global de gravité (5) dans le plan de la plaque cible (2),
- des moyens (21) pour combiner à ladite image radiographique numérisée du patient l'image (Hg) numérisée de l'ombre portée de l'axe global de gravité (5), et pour générer ainsi une image numérisée combinée.
- des moyens (23) pour visualiser ladite image numérisée carabinée,
et **en ce que** les moyens pour générer une image numérisée de l'ombre portée de l'axe global de gravité comprennent :
- une unité de calcul (21) et une mémoire (24) associée dans laquelle sont enregistrées des données géométriques correspondant aux positions relatives de la source de rayons X (1), du plan de la plaque sensible (2), et des moyens supports de patient (3) dans un repère fixe (XbObYb),
- un programme enregistré dans une zone de mémoire de programme (26) de la mémoire (24), adapté pour mémoriser lesdits signaux de position à l'instant de prise d'image radiographique, et adapté pour calculer en fonction desdits signaux de position la droite (Hg) intersection du plan de la plaque sensible (2) et du plan vertical passant par la source de rayons X (1) et par la position horizontale (Og) de l'axe global de gravité (5), et pour mémoriser le résultat de ce calcul qui constitue l'image de l'axe global de gravité.

2. Dispositif selon la revendication 1, **caractérisé en ce que** :
- il comprend un scanner (20) pour scruter par balayage l'image radiographique réalisée sur la plaque cible (2) sensible aux rayons X et pour produire une suite de signaux numériques constituant l'image radiographique numérisée,
- le programme est adapté pour assurer la réception desdits signaux numériques et pour les enregistrer dans la mémoire (24),
- le programme est adapté pour modifier l'image radiographique numérisée enregistrée en substituant l'image de l'axe global de gravité (Hg), générant ainsi ladite image numérisée combinée,
- et le programme est adapté pour afficher ladite image numérisée combinée sur un moniteur (23), ou pour produire son impression sur un support.

3. Dispositif selon l'une des revendications 1 ou 2, **caractérisé en ce que** le programme comprend une séquence de calibrage, adaptée pour scruter l'image radiographique d'un objet de calibrage (40) à repères radio-opaques (A, B, C, D, E, F, G, H) et pour calculer, à partir des dimensions et positions connues des repères radio-opaques dudit objet de calibrage (40), lesdites données géométriques, et pour les enregistrer dans la mémoire (24).

4. Dispositif selon l'une quelconque des revendications 1 à 3, **caractérisé en ce qu'**il comprend :
- des moyens supports de patient (3) ayant un plateau de force (15) de balance modifiée porté par une pluralité de capteurs de force (C1, C2, C3) répartis selon des positions repérées par rapport au repère (XbObYb) et produisant des signaux de force utilisés par l'unité de calcul et le programme comme signaux de position,
- un poids d'étalonnage amovible et positionnable sur les moyens supports de patient (3), ayant une forme déterminée, ayant des moyens de repérage de position à mettre en coïncidence avec des moyens de repérage complémentaires des moyens supports de patient (3), et ayant un centre de gravité de position relative connue par rapport aux moyens de repérage de position,
- le programme enregistré conprenant une séquence d'étalonnage adaptée pour calculer les paramètres de correction des signaux de force produits par chaque capteur (C1, C2, C3) de façon à amener en coïncidence le centre de gravité calculé avec la position géométrique connue du centre de gravité réel du poids d'étalonnage, les paramètres de correction étant alors enregistrés en mémoire pour permettre les calculs ultérieurs fiables de position de l'axe global de gravité des patients.

5. Dispositif selon l'une quelconque des revendications 1 à 4, **caractérisé en ce que** ledit repère fixe (XbObYb) est centré (Ob) sur le plan (XOZ) vertical passant par la source de rayons X (1) et perpendiculaire aux moyens supports de plaque cible (2) sensible aux rayons X.

6. Dispositif selon l'une quelconque des revendications 1 à 4, **caractérisé en ce qu'**il est agencé pour prendre une radiographie de la zone thoracique, de la zone lombaire, de la zone pelvienne et de la zone fémorale supérieure du patient (4).

7. Dispositif selon l'une quelconque des revendications 1 à 6, **caractérisé en ce que** les moyens supports de patient (3) sont montés rotatifs autour d'un axe vertical médian (10), permettant d'orienter angulairement le patient (4) par rapport à la direction de propagation des rayons entre la source de rayons x (1) et les moyens supports de plaque cible (2).

8. Dispositif selon la revendication 7, **caractérisé en ce que** les moyens supports de patient (3) comprennent des appuis-bras (8, 9) sur lesquels le patient (4) peut appuyer ses bras (6, 7) dans une position définie avancée.

9. Dispositif selon la revendication 8, **caractérisé en ce que** les appuis-bras (8, 9) sont solidaires d'un plateau tournant (13) qui lui-même porte un plateau de force (15) de balance modifiée par une pluralité de capteurs de force (C1, C2, C3) répartis selon des positions repérées par rapport au repère (XbObYb) et produisant des signaux de force utilisés par l'unité de calcul et le programme comme signaux de position.

## Patentansprüche

1. Einrichtung zur Auswertung des Gleichgewichtstandes des menschlichen Körpers, umfassend eine Röntgenstrahlenquelle (1) und Haltemittel für die röntgenstrahlempfindliche Zieltafel (2), um auf der Zieltafel (2) ein Röntgenbild des Patienten herzustellen, Patientenlagerungsmittel (3), die so ausgelegt sind, dass sie einen Patienten (4) in einer fixen Position zwischen der Röntgenstrahlenquelle (1) und den Haltemitteln der Zieltafel (2) halten und Positionssignale erzeugen, welche die horizontale Position der globalen Schwerkraftachse (5) des Patienten (4) in Bezug zu der Röntgenstrahlenquelle (1) und der Zieltafel (2) darstellen,
**dadurch gekennzeichnet, dass** sie Folgendes umfasst:
- Mittel (20), um das auf der Zieltafel (2) hergestellte Röntgenbild des Patienten durch Abtastung zu digitalisieren, und um **dadurch** ein digitalisiertes Röntgenbild zu erzeugen,
- Mittel (21), um in Abhängigkeit von den Positionssignalen ein digitalisiertes Bild des Schattenwurfs (Hg) der globalen Schwerkraftachse (5) in der Ebene der Zieltafel (2) zu erzeugen,
- Mittel (21), um das digitalisierte Röntgenbild des Patienten mit dem digitalisierten Bild (Hg) des Schattenwurfs der globalen Schwerkraftachse (5) zu kombinieren, und um **dadurch** ein kombiniertes digitalisiertes Bild zu erzeugen, und
- Mittel (23), um das kombinierte digitalisierte Bild zu visualisieren,
und dass die Mittel zum Erzeugen eines digitalisierten Bildes des Schattenwurfs der globalen Schwerkraftachse Folgendes umfassen:
- eine Rechnereinheit (21) und einen zugehörigen Speicher (24), in dem geometrische Daten gespeichert werden, die den relativen Positionen der Röntgenstrahlenquelle (1), der Ebene der empfindlichen Tafel (2) und den Patientenlagerungsmitteln (3) in einem fixen Koordinatensystem (XbObYb) entsprechen,
- ein Programm, das in einem Bereich des Programmspeichers (26) des Speichers (24) gespeichert wird, das so ausgelegt ist, dass es die Positionssignale zum Zeitpunkt der Aufnahme des Röntgenbilds speichert, und das so ausgelegt ist, dass es in Abhängigkeit von den Positionssignalen die Schnittgerade (Hg) der Ebene der empfindlichen Tafel (2) und der vertikalen Ebene, die durch die Röntgenstrahlenquelle (1) und durch die horizontale Position (Og) der globalen Schwerkraftachse (5) hindurchführt, berechnet, und das Ergebnis dieser Berechnung, welches das Bild der globalen Schwerkraftachse darstellt, speichert.

2. Einrichtung nach Anspruch 1,
**dadurch gekenrizeichnet,** dass:
- es einen Scanner (20) umfasst, um das Röntgenbild, das auf der röntgenstrahlempfindlichen Zieltafel (2) erzeugt wird, durch Abtastung zu scannen, und um eine Abfolge von digitalen Signalen zu erzeugen, die das digitalisierte Röntgenbild darstellen,
- das Programm so ausgelegt ist, dass es den Empfang der digitalen Signale sicherstellt und sie in dem Speicher (24) speichert,
- das Programm so ausgelegt ist, dass es das digitalisierte gespeicherte Röntgenbild ändert, indem es das Bild der globalen Schwerkraftachse (Hg) ersetzt und **dadurch** das kombinierte digitalisierte Bild erzeugt,
- und das Programm so ausgelegt ist, dass es das kombinierte digitalisierte Bild auf einem Monitor (23) anzeigt oder es auf einen Träger ausdruckt.

3. Einrichtung nach einem der Ansprüche 1 oder 2,
**dadurch gekennzeichnet,**
**dass** das Programm eine Kalibrierungssequenz umfasst, die so ausgelegt ist, dass sie das Röntgenbild eines Kalibrierungsobjekts (40) mit röntgenfähigen Markierungen (A, B, C, D, E, F, G, H) scannt und anhand von bekannten Abmessungen und Positionen der röntgenfähigen Markierungen des Kalibrierungsobjekts (40) die geometrischen Daten berechnet und sie in dem Speicher (24) speichert.

4. Einrichtung nach einem der Ansprüche 1 bis 3,
**dadurch gekennzeichnet,**
**dass** sie Folgendes umfasst:
- Patientenlagerungsmittel (3) mit einer Kraftmessplatte (15) für Gleichgewichtsänderungen, die von einer Vielzahl von Kraftsensoren (C1, C2, C3) getragen wird, die entsprechend den in Bezug zu dem Koordinatensystem (XbObYb) gesetzten Positionen verteilt sind, und Kraftsignale erzeugt, die von der Rechnereinheit und dem Programm als Positionssignale verwendet werden,
- ein abnehmbares und auf den Patientenlagerungsmitteln (3) positionierbares Kalibriergewicht von vorbestimmter Form mit Positionsortungsmitteln, die mit passenden Ortungsmitteln der Patientenlagerungsmittel (3) in Übereinstimmung gebracht werden müssen, und mit einem Schwerpunkt, dessen relative Position in Bezug zu den Positionsortungsmitteln bekannt ist,
- das gespeicherte Programm mit einer Kalibrierungssequenz, die so ausgelegt ist, dass sie die Korrekturparameter der Kraftsignale, die von dem jeweiligen Sensor erzeugt werden (C1, C2, C3), berechnet, so dass der berechnete Schwerpunkt mit der bekannten geometrischen Position des tatsächlichen Schwerpunkts des Kalibriergewichts in Übereinstimmung gebracht wird, wobei daraufhin die Korrekturparameter im Speicher gespeichert werden, um die späteren zuverlässigen Berechnungen der Position der globalen Schwerkraftachse der Patienten zu erlauben.

5. Einrichtung nach einem der Ansprüche 1 bis 4,
**dadurch gekennzeichnet,**
**dass** das feste Koordinatensystem (XbObYb) auf der vertikalen Ebene (XOZ), die durch die Röntgenstrahlenquelle (1) hindurchgeht, zentriert (Ob) und senkrecht zu den Haltemitteln der röntgenstrahlempfindlichen Zieltafel (2) ist.

6. Einrichtung nach einem der Ansprüche 1 bis 4,
**dadurch gekennzeichnet,**
**dass** sie so angeordnet ist, dass sie eine Röntgenaufnahme des Brustkorbbereichs, des Lendenbereichs, des Beckenbereichs und des oberen Oberschenkelbereichs des Patienten (4) erstellt.

7. Einrichtung nach einem der Ansprüche 1 bis 6,
**dadurch gekennzeichnet,**
**dass** die Patientenlagerungsmittel (3) drehbar um eine vertikale Mittelachse (10) montiert sind und es erlauben, den Patienten (4) in Bezug zu der Ausbreitungsrichtung der Strahlen zwischen der Röntgenstrahlenquelle (1) und den Haltemitteln der Zieltafel (2) winklig auszurichten.

8. Einrichtung nach Anspruch 7,
**dadurch gekennzeichnet,**
**dass** die Patientenlagerungsmittel (3) Armlehnen (8, 9) umfassen, auf die der Patient (4) seine Arme (6, 7) in einer definierten nach vorne gerichteten Position aufstützen kann.

9. Einrichtung nach Anspruch 8,
**dadurch gekennzeichnet,**
**dass** die Armlehnen (8, 9) einstückig mit einer Drehplatte (13) ausgebildet sind, die wiederum eine Kraftmessplatte (15) für Gleichgewichtsänderungen trägt, die von einer Vielzahl von Kraftsensoren (C1, C2, C3) getragen wird, die entsprechend den in Bezug zu dem Koordinatensystem (XbObYb) gesetzten Positionen verteilt sind, und Kraftsignale erzeugt, die von der Rechnereinheit und dem Programm als Positionssignale verwendet werden.

## Claims

1. A device for evaluating a balanced position of the human body, comprising an X-ray source (1) and X-ray sensitive target plate support means (2), for producing on the target plate (2) a radiographic image of the patient, and patient support means (3) adapted for supporting a patient (4) in a fixed position between the X-ray source (1) and the target plate support means (2) and for generating image position signals for the horizontal position of the overall gravity axis (5) of the patient (4) relative to the X-ray source (1) and the target plate (2),
**characterised in that** it comprises:
- means (20) for digitising by scanning the radiographic image of the patient produced on the target plate (2) and for thereby generating a digitised radiographic image,
- means (21) for generating, as a function of said position signals, a digitised image of the shadow (Hg) cast by the overall gravity axis (5) in the plane of the target plate (2),
- means (21) for combining with said digitised radiographic image of the patient the digitised image (Hg) of the shadow cast by the overall gravity axis (5) and for thereby generating a combined digitised image,
- means (23) for displaying said combined digitised image,
and **in that** the means for generating a digitised image of the shadow cast by the overall gravity axis, comprise:
- an arithmetic unit (21) and an associated memory (24) in which are recorded geometric data corresponding to the relative positions of the X-ray source (1), the plane of the sensitive plate (2), and the patient support means (3) in a fixed system of coordinates (XbObYb),
- a program recorded in a program memory zone (26) of the memory (24), adapted for storing said position signals at the instant at which the radiographic image is taken, and adapted for calculating as a function of said position signals the intersection straight line (Hg) of the plane of the sensitive plate (2) and the vertical plane passing through the X-ray source (1) and through the horizontal position (Og) of the overall gravity axis (5), and for storing the result or said calculation, which constitutes the image of the overall gravity axis.

2. A device according to claim 1, **characterised in that**:
- it comprises a scanner (20) for analysing by scanning the radiographic image produced on the X-ray sensitive target plate (2) and for producing a series of digital signals constituting the digitised radiographic image,
- the program is adapted for ensuring reception of said digital signals and recording them in the memory (24),
- the program is adapted for modifying the recorded digitised radiographic image by substituting the image of the overall gravity axis (Hg), thus generating said combined digitised image,
- and the program is adapted for displaying said combined digitised image on a monitor (23), or for printing it out on a substrate.

3. A device according to one of claims 1 or 2, **characterised in that** the program comprises a calibration sequence, adapted for analysing the radiographic image of a calibration object (40) with radiopaque reference points (A, B, C, D, E, F, G, H) and for calculating said geometric data from the known dimensions and positions of the radiopaque reference points of said calibration object (40) and recording them in the memory (24).

4. A device according to any one of claims 1 to 3, **characterised in that** it comprises :
- patient support means (3) having a force table (15) of a modified set of scales, said table being borne by a plurality of force sensors (C1, C2, C3) distributed in accordance with positions located relative to the system of coordinates (XbObYb) and producing force signals used by the arithmetic unit and the program as position signals,
- a calibration weight movable and positionable on the patient support means (3), having a determined shape, having position locating means to be brought into coincidence with complementary locating means of the patient support means (3), and having a centre of gravity with a known relative position relative to the position locating means,
- the recorded program comprising a calibration sequence adapted for calculating the correction parameters of the force signals produced by each sensor (C1, C2, C3) in such a way as to bring into coincidence the calculated centre of gravity with the known geometric position of the real centre of gravity of the calibration weight, the correction parameters then being recorded in the memory to allow subsequent reliable calculations of the position of the overall gravity axis of the patients.

5. A device according to any one of claims 1 to 4, **characterised in that** said fixed system of coordinates (XbObYb) is centred (Ob) on the vertical plane (XOZ) passing through the X-ray source (1) and perpendicular to the X-ray sensitive target plate support means (2).

6. A device according to any one of claims 1 to 4, **characterised in that** it is arranged to take a radiograph of the thoracic zone, the lumbar zone, the pelvic zone and the upper femoral zone of the patient (4).

7. A device according to any one of claims 1 to 6, **characterised in that** the patient support means (3) are mounted so as to be rotatable about a median vertical axis (10), allowing angular orientation of the patient (4) relative to the direction of propagation of the rays between the X-ray source (1) and the target plate support means (2).

8. A device according to claim 7, **characterised in that** the patient support means (3) comprise armrests (8, 9) on which the patient (4) may rest his/her arms (6, 7) in a defined forward position.

9. A device according to claim 8, **characterised in that** the armrests (8, 9) are integral with a revolving platform (13), which itself carries a force table (15) of a set of scales modified by a plurality of force sensors (C1, C2, C3) distributed in accordance with positions located relative to the system of coordinates (XbObYb) and producing force signals used by the arithmetic unit and the program as position signals.
